# EUROPEAN PATENT APPLICATION

(11) **EP 4 704 101 A1**
(43) Date of publication of application: **04.03.2026**
(21) Application number: 24197014.4
(22) Date of filing: 28.08.2024
(51) Int. Cl.: G16H 30/20, G16H 30/40

(54) **DEVICE AND METHOD FOR ORGAN POSITIONING ASSESSMENT**

(71) Applicant: Siemens Healthineers AG, 91301 Forchheim (DE)
(72) Inventor: Biniazan, Ramyar, 90402 Nürnberg (DE); Fieselmann, Andreas, 91052 Erlangen (DE)
(74) Representative: Siemens Healthineers Patent Attorneys

(57) **Abstract**

The invention pertains to a device (10) for organ positioning assessment for the acquisition of images (I) in the course of a medical examination of a patient (P), comprising:
- a data-interface (11) designed for receiving and/or automatically retrieving physical patient-data (D),
- a pre-assessment-unit (12) designed for generating a positioning-advice (A) for the medical examination based on the type of medical examination and the provided patient-data (D),
- a data-interface (11) designed for outputting the positioning-advice (A),
- a data-interface (11) designed for receiving and/or automatically retrieving a number of examination-images (I) of the patient (P),
- a post-assessment-unit (13) designed for generating patient-specific feedback-data (F) based at least on the number of examination-images (I) and the positioning-advice (A),
- a data-interface (11) designed for outputting the feedback-data (F).

Furthermore, the invention describes a method and a medical imaging system.

## Description

The invention describes a device and a method for organ positioning assessment for the acquisition of images in the course of a medical examination of a patient as well as a medical imaging system.

Correct organ positioning is crucial for many kinds of medical examinations. For example, in order to receive optimal X-ray mammograms, the breast must be positioning correctly. However, also for other examinations, e.g. of the chest, the knee or the head, the respective body-part must be arranged correctly, in order to have a good view on the region of interest.

Looking at the exemplary examination of breast cancer screening, the positioning of the breast must follow certain established guidelines. If the breast is not positioned well, the mammogram may not be suitable for diagnostic purposes. However, an ideal breast positioning (according to the general guideline) may not always be feasible due to patient-individual constraints, e.g. body weight, disabilities or physical constraints.

The technical problem appears that there is currently only a general support but no patient-specific (e.g. individual constraints or physical inabilities) and/or radiographer-specific (e.g. the level of experience) positioning support available for a mammographic examination. This also appears for other kinds of examinations.

Current solutions for assessment of breast positioning (e. g. TruPGMI, Volpara Health) are based on analyzing the just acquired mammography images. However, such solutions have limitations. They assess each image based on a general guideline and they don't consider patient constraints and also not the radiographers' level of experience. In addition, they can only be used after acquisition, therefore possible mispositioning cannot be avoided.

It is the object of the present invention to improve the known systems and methods and provide a device and a method for organ positioning assessment for the acquisition of images in the course of a medical examination of a patient as well as a medical imaging system, for overcoming the above described problems. Especially, the object of the present invention is to give a radiographer the optimal information for correct patient positioning.

This object is achieved by a device according to claim 1, a method according to claim 5 and a medical imaging system according to claim 13.

A device according to the invention serves for organ positioning assessment for the acquisition of images in the course of a medical examination of a patient. It comprises:
- a data-interface designed for receiving and/or automatically retrieving physical patient-data,
- a pre-assessment-unit designed for generating a positioning-advice for the medical examination based on the type of medical examination and the provided patient-data,
- a data-interface designed for outputting the positioning-advice,
- a data-interface designed for receiving and/or automatically retrieving a number of examination-images of the patient,
- a post-assessment-unit designed for generating patient-specific feedback-data based at least on the number of examination-images and the positioning-advice
- a data-interface designed for outputting the feedback-data.

The invention deals with organ positioning assessment for the acquisition of images in the course of a medical examination of a patient. It is very advantageous for X-ray imaging, e.g. fluoroscopy or radiography, but also for MRT-examinations or ultrasound examinations.

The device comprises a pre-assessment-unit, a post-assessment-unit and a number of data-interfaces that could be multiple data-interfaces or one single data-interface with multiple functionalities or multiple connections. There could be a data-interface for receiving data and another data-interface for sending data or there could be a data-interface for the pre-assessment-unit and another data-interface for the post-assessment-unit.

The (or one) data-interface is designed for receiving and/or automatically retrieving physical patient-data. This physical patient-data could include patient-data about physical inabilities or malformations of the patient, but also physical issues concerning prior exams of this patient, e.g. re-positioning, special positioning.

The pre-assessment-unit is designed for generating a positioning-advice for the medical examination based on the type of medical examination and the provided patient-data. The positioning-advice may be an advice for the required body posture of the patient as well as information about what special issues should be respected (e.g. "caution: broken arm").

Since this advice is individual for different kinds of examinations, the positioning-advice depends on the type of medical examination (e.g. mammography, examination of the chest or knee). Furthermore, the positioning-advice depends on abilities and disabilities of the patient. Thus, the positioning-advice is also based on the provided patient-data. In the case the patient has a special physical inability (e.g. wheelchair bound), this has to be respected for the applicable and possible body posture.

The (or one) data-interface is designed for outputting the positioning-advice. This could be done on a display, especially graphically, but also in form of control commands to control motors of an automatic positioning device. The position advice could comprise information about a center position of the body in addition with rotation angles around body axes and/or the position of extremities relative to a body-region.

These units are used for a first analysis (and possibly for positioning) before the actual examination. The following units are used for a second analysis and for producing feedback information.

The (or one) data-interface is designed for receiving and/or automatically retrieving a number of examination-images of the patient. These examination-images could be directly acquired during an examination or downloaded from a database after the examination. These examination-images are preferably recorded with the positioning-advice given by the units explained above. However, they could e.g. also be recorded in order to check other positioning-advices.

The post-assessment-unit is designed for generating patient-specific feedback-data based at least on the number of examination-images and the positioning-advice (and especially also the patient-data). The feedback-data should give a feedback based on the quality of the image about the positioning advice. This feedback-data could be used to ameliorate the device (in order to train machine-learning models used by the device), to train a user or as background information for an examiner. The feedback-data could comprise information about how a positioning-advice has been implemented, about the quality of a positioning-advice or for comparison of positioning-advices. It is preferred to additionally use the patient data in order to evaluate the condition of the positioning-advice for the feedback data. For example, when a patient is wheelchair-bound, the last positioning-advices could be used in order to evaluate whether the images of the actual positioning-advice are better or not compared to the images recorded with other positioning-advices.

The (or one) data-interface is designed for outputting the feedback-data. This could be displayed to a user, used for training or for evaluation issues.

A method according to the invention serves for organ positioning assessment for the acquisition of images in the course of a medical examination of a patient, especially with a device according to the invention. It comprises the following steps:
- providing physical patient-data about the patient and/or prior exams of this patient,
- generating a positioning-advice for the medical examination based on the type of medical examination and the provided patient-data,
- outputting the positioning-advice and using it to position the patient for the medical examination and acquire a number of examination-images of the patient,
- providing an examination-image of the patient,
- generating patient-specific feedback-data based at least on the number of examination-images and the positioning-advice,
- outputting the feedback-data.

First, physical patient-data about the patient and/or prior exams of this patient are provided. As already indicated above, this data should give information about the physical abilities of the patient concerning the examination. For example, the data may give at least information about the body part to be examined (e.g. known condition of a breast for mammography), but preferably also about the patient (size, weight, age). It is preferred that the physical data comprises information about inabilities (e.g. "wheelchair bound", "broken arm", "uncooperative", "panics in small rooms"). However, also data about prior exams could be very helpful, like positioning for these exams or failures due to mispositioning. In short, any information that could be used for an (optimal) positioning of the patient could be included in the physical data.

The positioning-advice is preferably generated automatically. This could be done by a machine-learning model trained with physical data of the patient and information about the examination and an information about the optimal positioning as ground truth. However, this could also be done by a conventional algorithm that links keywords of the patient data with positions given in a list for a certain type of examination. There could be several lists for several types of examination.

As already said above, this positioning-advice is outputted (e.g. on a screen for a user or as control commands for an automatic positioning system) and used it to position the patient for the medical examination and acquire a number of examination-images of the patient.

Then the images are acquired. This well-known step is not inadequately needed for the method, but the images acquired should be used. However, it is not necessary to position the patient as suggested by the method. Since the user is free to follow the positioning advice or not, it could also be examined what happens, if a user should ignore the suggestions of the method. It follows the second analysis producing a feedback.

The first step of this second analysis is the provision of an examination-image of the patient, preferably the examination-images where the positioning advice has been followed.

Now, patient-specific feedback-data is generated based at least on the number of examination-images and the positioning-advice (and especially also the patient-data). This could be done in an automatic manner by an algorithm designed to search the examination-images for special structures and compare the structures with a given list of structures. In the case all structures have been found, the feedback is good, in the case there are structures missing or deformed, the feedback is not good. The search may be done by a machine-learning model trained to segment images and search for structures in these segments.

Then, the feedback-data is outputted as already explained above.

In a practical approach the method may be realized as explained in the following. The device or the method provides advice and feedback to a radiographer about positioning, e.g. of a breast, at two time points: prior and after an X-ray examination. The system uses available data from the patient which comprises at least one of the following information:
- data about prior exams, especially mammograms, (e.g. retrieved from PACS) including their radiology reports,
- general patient information (such as age, BMI/weight, disabilities; retrieved from the hospital information system for example),
- situation information from the examination room (this could be a room camera for example, that records information about the patient's compliance during the examination).

At the first time point (prior to the exam), the available patient-data about the patient is used to provide the radiographer with patient-specific positioning advice. At the second time point (after the exam), the just acquired image and the available data are used to provide the radiographer with feedback. This feedback can include an assessment of the just acquired images, especially mammogram, (is there any positioning deficiency that could have been avoided?) and- if a positioning deficiency has been found - it can also include feedback for the radiographer on how to avoid this deficiency in the future.

Assuming all physical patient-data has been retrieved, several analyses can be made to give advice to the radiographer for (e.g. breast) positioning before the examination. These analyses can include:
- Parsing of radiology reports (did the radiologists mention non-optimal positioning in the past?)
- Analyzing all available information about the patient (BMI/weight, disabilities, ...) and comparing those to a database of known positioning challenges
- Analyzing (possibility / willingness of) compliance of the patient based on room camera video recording.

In addition, the pixel data of prior images, e.g. mammograms, can be analyzed. There are state of the art methods to assess patient positioning according to general guidelines on mammographic images, see Watanabe et al. "Quality control system for mammographic breast positioning using deep learning", Scientific Reports volume 13, Article number: 7066; 2023) for an example.

In the special case that when the prior images, e.g. mammograms, have been acquired by the same radiographer that is going to take the current image, then the analysis of the prior mammogram's pixel data could be used to give radiographer-specific advice.

The system may identify a mispositioning in the current acquired image. However, it is known from clinical reality that some kinds of mispositioning are difficult to avoid in a current exam situation.

There could be cases where optimal positioning is not possible, e.g. for a mammography where perfect positioning (according to the definitions in the guideline) is impossible due to surgery and having surgical clips on breast. In such cases having comparable breast size between two views is not possible. Furthermore, it could be possible that due to the pain of the patient, avoiding skin folds is also not possible.

The present invention could also identify cases of mispositioning that are not avoidable. The term "avoidable" means that this mispositioning would not be present when another person skilled in using state of the art breast positioning techniques would have made the exam.

Formally, avoidability X can be defined as X = percentage of radiographers of a reference group that can perform the positioning without the deficiency present.

The avoidability X can range from 0% (no radiographer would be able to avoid it) to 100% (all radiographers from the reference group would avoid it). It could then be classified into certain classes (or bins), for example:
0% <= X <= 5% means almost impossible to avoid
5% < X <= 25% difficult to avoid
25% < X <= 75% generally avoidable
75% < X <= 100% clearly avoidable.

A method to automatically quantify avoidability can be used to make a supervised training of a machine-learning model. During training the model takes the (current) image's pixel data and meta-information as input and is trained by radiographers' expert opinion as output.

When a deficiency has been found that was classified as at least generally avoidable then the software can provide the radiographer with detailed feedback on how to avoid such deficiencies in the future. This information can come from a well-curated and edited database of domain expert's opinion on how to avoid certain types of positioning deficiencies.

A medical imaging system according to the invention is especially a mammography-system or a fluoroscopy-system, and comprises a device according to the invention and/or it is designed for performing a method according to the invention.

Some units or modules of the invention mentioned above can be completely or partially realized as software modules running on a processor of a computing system. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the methods, at least those steps that could be executed by a computer, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

A preferred embodiment of the device comprises a classification-unit, preferably as part of the post-assessment-unit, wherein the classification-unit is designed to determine a mispositioning of the patient in the number of examination-images, and to determine, whether the mispositioning was avoidable or not.

Here it should be noted that mispositioning is basically a state. This state is quantified by the displacement for example. So avoidability refers to the state in the first place but could also be linked to the value that is a measure for the state. The term "avoidable" means that this mispositioning would not be present when another person skilled in the art (e.g. of breast positioning techniques) would have made the exam. For example, regarding a lateral mispositioning avoidability could be defined by the grade of mispositioning. A mispositioning of a mm could then be inavoidable, a mispositioning of a cm could be difficult to avoid but a mispositioning of a dm would be avoidable. But there may also be avoidabilities that could not be given by measures, for example, confuse left with right would be generally avoidable.

In general, by using an AI model, of course this is rather task-specific. A preferred approach is creating a data set containing images, e.g. mammograms, with certain positioning deficiencies and expert annotations that say based on the expert's opinion if that deficiency could have been avoided by proper positioning given by the state of the art.

It is preferred that the classification-unit is a machine-learning model trained to segment regions in the number of examination-images that show irregularities due to mispositioning and allocate the segmented regions to a list of multiple mispositioning-types. It could e.g. be a mispositioning if all the desired landmarks or structures could not be seen in the images.

A preferred embodiment of the device wherein the classification-unit is designed to parse image-data of earlier examinations and determine an avoidability-value for a mispositioning based on an output of a machine-learning model trained on experts' opinions or on a ratio of occurrences of a number of mispositioning-types of the list compared to the total number of images. Instead of parsing image data, this data could also be parsed in an early stage by another unit. In this case, the device could comprise a list of avoidability-values for certain mispositioning-types.

Preferably, the classification unit is designed to bin the avoidability-values of a number of mispositioning-types into certain predefined value-bins in order to allocate avoidability-statements to the mispositioning-types based on the bins. Such statements could be: "almost impossible to avoid", "difficult to avoid", "generally avoidable" and "clearly avoidable".

A preferred embodiment of the device of the preceding claims, wherein the data-interface designed for receiving and/or automatically retrieving physical patient-data and/or prior exams of this patient is designed to receive data via a Picture Archiving and Communication System PACS and/or a hospital information system HIS and/or a Radiology Information System RIS and/or sensors, especially cameras.

Regarding a preferred method, the patient-data is general patient information, such as e.g. age, BMI/weight, disabilities, alterations of organs like clips in a breast. The general patient information may be retrieved from the hospital information system (HIS) or being manually inputted. Alternatively or additionally it is preferred that the patient-data of a patient comprises data about prior exams of this patient, especially data about prior examinations similar to the intended medical examination and/or situation information from the examination room. This could be a room camera for example, that records information about the patient's compliance during the examination.

Regarding a preferred method, for generating the patient-specific positioning-advice the patient-data is analyzed, wherein this analysis includes one or more of the steps:
- parsing of radiology reports, e.g. based on the question "did the radiologists mention non-optimal positioning in the past?",
- analyzing general patient information (e.g. BMI/weight, disabilities or unwillingness) and comparing those to a database of known positioning challenges,
- analyzing compliance of the patient based on room camera video recording,
- analyzing pixel data of images of prior exams of the patient, (see above mentioned state of the art methods).

Preferably it is determined, whether the person performing the intended medical examination has performed a similar examination on the patient and a person specific advice is generated based on the respective prior examination and added to the positioning-advice.

Thus, there is the possibility to analyze the history of the patient in order to get an overview about the optimal positioning, as well as the possibility to analyze the history of the technician performing the examination in order to get an overview about the optimal explanations for positioning.

Preferably, the intended examination is a mammography or a fluoroscopy or a CT-examination or an MRI-examination or a radiography examination or an ultrasound examination. The positioning method may be used for several different examinations that may not always be X-ray examinations. However, since X-ray examinations are always connected with a dose applied to a patient, the method is especially advantageous to prevent defective X-ray images.

According to a preferred method, for creating the feedback-data the number of examination-images is automatically assessed including concerning the quality of positioning. The assessment preferably comprises a search for a number and/or a grade of positioning deficiencies. It is preferred that the feedback-data also includes automatically generated notes concerning methods how to avoid a positioning deficiency in the future.

Preferably, the number of examination-images is analyzed by looking for a mispositioning, determining whether the mispositioning was avoidable or not. As said above, the term "avoidable" means that this mispositioning would not be present when another person skilled in using state of the art breast positioning techniques would have made the exam.

It is preferred that an avoidability-value is calculated as a percentage of radiographers that can perform the positioning without the mispositioning. Here as said above, the avoidability can be from 0% (no radiographer would be able to avoid it) to 100% (all radiographers from the group would avoid it) and it could then be classified into several classes.

Preferably, avoidability-values of a number of mispositioning-types are binned into certain predefined value bins in order to allocate avoidability-states to the mispositioning-types based on the bins. These states may be "almost impossible to avoid", "difficult to avoid", "generally avoidable", "clearly avoidable".

Regarding a preferred method, where a mispositioning is found in an examination image, the method comprises the steps:
- assigning the mispositioning in the examination-image to a mispositioning-type of a predefined list,
- determining an avoidability for this mispositioning type based on an avoidability-value or an avoidability-statement,
- comparing the avoidability with a predefined threshold-value indicating whether the mispositioning-type is avoidable or not,
- in the case the comparison shows that the mispositioning-type is avoidable, outputting a predefined dataset allocated to the mispositioning type comprising detailed information on how to avoid such mispositioning in the future. This information may come from a well-curated and edited database of domain expert's opinion on how to avoid certain types of positioning deficiencies.

Preferably, the positioning-advice is designed according to the physical nature of the patient, and is especially patient-specific, and/or is designed based on predefined information and/or prior data of the examiner for the medical examination.

The use of Al-based methods (Al: "artificial intelligence") is preferred for the method according to the invention. Artificial intelligence is based on the principle of machine-based learning and is usually carried out with an adaptive algorithm that has been trained accordingly. The expression "machine-learning" is often used for machine-based learning, which also includes the principle of "deep learning".

The methods may also include elements of "cloud computing". In the technical field of "cloud computing", an IT infrastructure is provided over a data-network, storage space or processing power and/or application software. The communication between the user and the "cloud" is achieved by means of data-interfaces and/or data transmission protocols. In the context of "cloud computing", in a preferred embodiment of the methods according to the invention, provision of data via a data channel (for example a data-network) to a "cloud" takes place. This "cloud" includes a (remote) computing system, e.g. a computer cluster that typically does not include the user's local machine. It is particularly preferred that the cloud service provides as well computing power as application software.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 shows a tomography-system (as example for an imaging system),
Figure 2 shows an example for an advice according to the invention,
Figure 3 shows a block diagram of the method according to the invention,
Figure 4 shows a practical application of the invention.

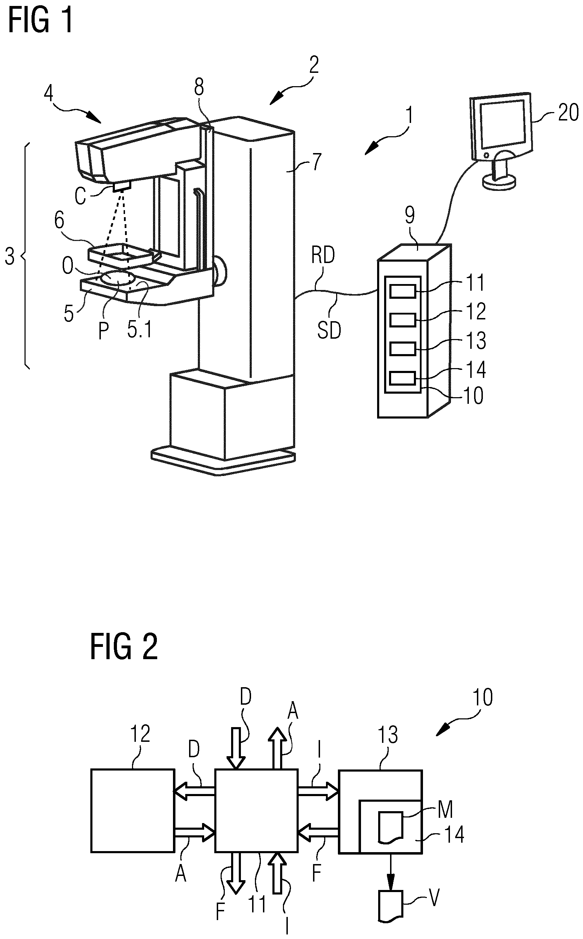

In Figure 1, a mammography system 1 in the form of a tomosynthesis system 1 is roughly schematically shown as an example for a medical imaging system. Relative directional information such as "top", "bottom" etc. refers to a tomosynthesis system 1 that has been set up for operation as intended. The tomosynthesis system 1 includes a tomosynthesis device 2 and a control device 9.

The tomosynthesis device 2 has a column 7 and a source-detector arrangement 3, which in turn include an X-ray emitter 4 and a detector 5 with a detector surface 5.1. The column 7 stands on the ground during operation. The source-detector arrangement 3 is slidably connected to it, so that the height of the detector surface 5.1, i.e. the distance to the ground, can be adjusted to a patient's chest height.

A breast O of the patient (shown here schematically) lies on the top side of the detector surface 5.1 as an examination object O to be examined. A compression plate 6 is arranged above the breast O and the detector surface 5.1, which is slidably connected to the source-detector arrangement 3. For the examination, the breast O is compressed and at the same time fixed by lowering the compression plate 6 onto it, so that pressure is exerted on the breast O between the compression plate 6 and the detector surface 5.1.

The X-ray emitter 4 is arranged and designed opposite the detector 5 in such a way that the detector 5 detects X-ray radiation R emitted by it after at least part of the X-ray radiation R has penetrated the patient's breast O. The X-ray emitter 4 can be pivoted relative to the detector 5 by means of a rotating arm 8 in a range of ± 50° about a basic position in which it is vertically above the detector surface 5.1. The section to be recorded can be specified or restricted using a collimator C.

The control device 9 receives the raw data RD of the measurement and sends control data SD to the tomosynthesis device 2 using a data interface. It is connected to a terminal 20 through which a user can communicate commands to the tomosynthesis system 1 or retrieve measurement results. The control device 9 can be arranged in the same room as the tomosynthesis device 2, but it can also be located in an adjacent control room or at an even further spatial distance
The control device 9 comprises a device 10 for organ positioning assessment for the acquisition of images I in the course of a medical examination of a patient P. The device comprises a data-interface 11, a pre-assessment-unit 12, a post-assessment-unit 13 and classification-unit 14.

Figure 2 shows an example for an advice according to the invention.

The data-interface 11 is designed for receiving and/or automatically retrieving physical patient-data D and outputting the positioning-advice A. Furthermore, the data-interface 11 is designed for receiving and/or automatically retrieving a number of examination-images I of the patient P and outputting the feedback-data F. The data-interface 11 could be designed for receiving and/or automatically retrieving physical patient-data D and/or prior exams of this patient P is designed to receive data via a Picture Archiving and Communication System PACS and/or a hospital information system HIS and/or a Radiology Information System RIS and/or sensors, especially cameras.

The pre-assessment-unit 12 is designed for generating a positioning-advice A for the medical examination based on the type of medical examination and the provided patient-data D.

The post-assessment-unit 13 is designed for generating patient-specific feedback-data F based at least on the number of examination-images I and the positioning-advice A.

The classification-unit 14 is designed to determine a mispositioning of the patient P in the number of examination-images I, and to determine, whether the mispositioning was avoidable or not. The classification-unit 14 may be a machine-learning model trained to segment regions in the number of examination-images I that show irregularities due to mispositioning and allocate the segmented regions to a list of multiple mispositioning-types M. The classification-unit 14 could be designed to parse image-data I of earlier examinations and determine an avoidability-value V for a mispositioning based on an output of a machine-learning model trained on experts' opinions or on a ratio of occurrences of a number of mispositioning-types M of the list compared to the total number of images I. The classification unit could also be designed to bin the avoidability-values V of a number of mispositioning-types M into certain predefined value-bins in order to allocate avoidability-statements to the mispositioning-types M based on the bins.

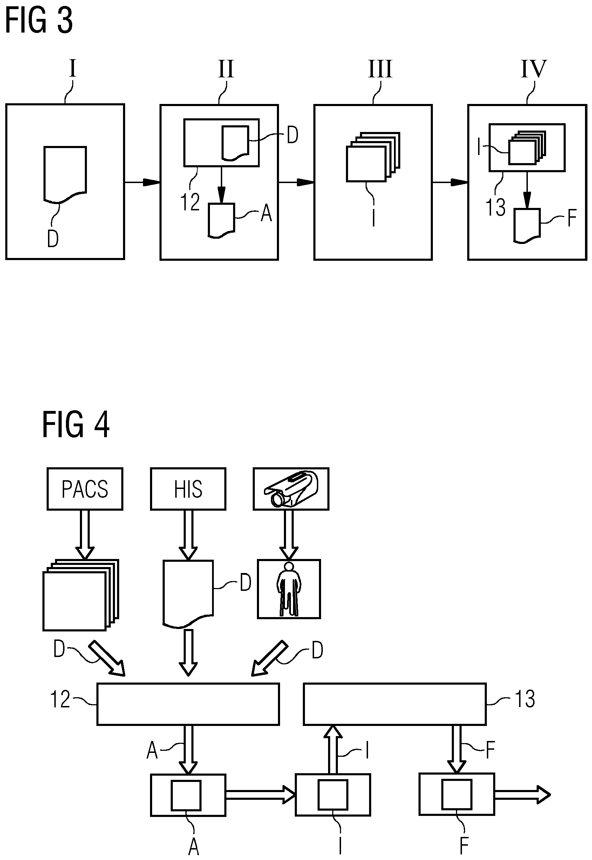

Figure 3 shows a block diagram of the method for organ positioning assessment for the acquisition of images I in the course of a medical examination of a patient P, especially with a device 10 as shown in figure 2.

In step I, physical patient-data D and/or prior exams of this patient P is provided.

In step II, a positioning-advice A for the medical examination is generated by the pre-assessment-unit 12 based on the type of medical examination and the provided patient-data D and outputted in order to use it to position the patient P for the medical examination and acquire a number of examination-images I of the patient P.

I step III, an examination-image I of the patient P is provided, e.g. after acquisition.

In step IV, patient-specific feedback-data F is generated by the post-assessment-unit 13 based at least on the number of examination-images I and the positioning-advice A and outputted.

Figure 4 shows a practical application of the invention. From above, physical patient-data D about the patient P (middle) and/or prior exams of this patient P (left) or the actual condition recorded with a camera (right) is provided.

This data is inputted in a pre-assessment-unit 12 and a positioning-advice A for the medical examination is generated and outputted (middle row).

The bottom row shows that the positioning-advice A is used to make an exam and acquire images I.

These images I are then inputted in a post-assessment-unit 13 and patient-specific feedback-data F is generated and outputted.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The expression "a number of" means "at least one". The mention of a "unit" or a "device" does not preclude the use of more than one unit or device. Independent of the grammatical term usage, individuals with male, female or other gender identities are included within the term.

## Claims

1. A device (10) for organ positioning assessment for the acquisition of images (I) in the course of a medical examination of a patient (P), comprising:
- a data-interface (11) designed for receiving and/or automatically retrieving physical patient-data (D),
- a pre-assessment-unit (12) designed for generating a positioning-advice (A) for the medical examination based on the type of medical examination and the provided patient-data (D),
- a data-interface (11) designed for outputting the positioning-advice (A),
- a data-interface (11) designed for receiving and/or automatically retrieving a number of examination-images (I) of the patient (P),
- a post-assessment-unit (13) designed for generating patient-specific feedback-data (F) based at least on the number of examination-images (I) and the positioning-advice (A),
- a data-interface (11) designed for outputting the feedback-data (F).

2. The device (10) according to claim 1, comprising a classification-unit (14), preferably as part of the post-assessment-unit (13), wherein the classification-unit (14) is designed to determine a mispositioning of the patient (P) in the number of examination-images (I), and to determine, whether the mispositioning was avoidable or not,
preferably wherein the classification-unit (14) is a machine-learning model trained to segment regions in the number of examination-images (I) that show irregularities due to mispositioning and allocate the segmented regions to a list of multiple mispositioning-types (M).

3. The device (10) according to claim 2, wherein the classification-unit (14) is designed to parse image-data (I) of earlier examinations and determine an avoidability-value (V) for a mispositioning based on an output of a machine-learning model trained on experts' opinions or on a ratio of occurrences of a number of mispositioning-types (M) of the list compared to the total number of images (I),
preferably wherein the classification unit is designed to bin the avoidability-values (V) of a number of mispositioning-types (M) into certain predefined value-bins in order to allocate avoidability-statements to the mispositioning-types (M) based on the bins.

4. The device (10) according to one of the preceding claims, wherein the data-interface (11) designed for receiving and/or automatically retrieving physical patient-data (D) and/or prior exams of this patient (P) is designed to receive data via a Picture Archiving and Communication System PACS and/or a hospital information system HIS and/or a Radiology Information System RIS and/or sensors, especially cameras.

5. A method for organ positioning assessment for the acquisition of images (I) in the course of a medical examination of a patient (P), especially with a device (10) according to one of the preceding claims, comprising the steps:
- providing physical patient-data (D) about the patient (P) and/or prior exams of this patient (P),
- generating a positioning-advice (A) for the medical examination based on the type of medical examination and the provided patient-data (D),
- outputting the positioning-advice (A) and using it to position the patient (P) for the medical examination and acquire a number of examination-images (I) of the patient (P),
- providing an examination-image (I) of the patient (P),
- generating patient-specific feedback-data (F) based at least on the number of examination-images (I) and the positioning-advice (A),
- outputting the feedback-data (F).

6. The method according to claim 5, wherein the patient-data (D) is general patient (P) information, and/or wherein the patient-data (D) of a patient (P) comprises data about prior exams of this patient (P), especially data about prior examinations similar to the intended medical examination and/or situation information from the examination room.

7. The method according to claim 5 or 6, wherein for generating the patient-specific positioning-advice (A) the patient-data (D) is analyzed, wherein this analysis includes one or more of the steps:
- parsing of radiology reports,
- analyzing general patient (P) information and comparing those to a database of known positioning challenges,
- analyzing compliance of the patient (P) based on room camera video recording,
- analyzing pixel data of images (I) of prior exams of the patient (P),
preferably wherein it is determined, whether the person performing the intended medical examination has performed a similar examination on the patient (P) and a person specific advice is generated based on the respective prior examination and added to the positioning-advice (A).

8. The method according to one of claims 5 to 7, wherein the intended examination is a mammogaphy or a fluoroscopy or a CT-examination or an MRI-examination or a radiography examination or an ultrasound examination.

9. The method according to one of claims 5 to 8, wherein for creating the feedback-data (F) the number of examination-images (I) is automatically assessed including concerning the quality of positioning, especially wherein the assessment comprises a search for a number and/or grade of positioning deficiencies,
preferably wherein the feedback-data (F) also includes automatically generated notes concerning methods how to avoid a positioning deficiency in the future.

10. The method according to one of claims 5 to 9, wherein the number of examination-images (I) is analyzed by looking for a mispositioning, determining whether the mispositioning was avoidable or not,
preferably wherein an avoidability-value (V) is calculated as a percentage of radiographers that can perform the positioning without the mispositioning,
preferably wherein avoidability-values (V) of a number of mispositioning-types (M) are binned into certain predefined value bins in order to allocate avoidability-states to the mispositioning-types (M) based on the bins,
preferably wherein the avoidability-value (V) for a mispositioning is based on an output of a machine-learning model trained on experts' opinions or on a ratio of occurrences of a number of mispositioning-types (M) of the list compared to the total number of images (I).

11. The method according to claim 10, wherein a mispositioning is found in an examination image (I), comprising the steps:
- assigning the mispositioning in the examination-image (I) to a mispositioning-type (M) of a predefined list,
- determining an avoidability for this mispositioning type based on an avoidability-value (V) or an avoidability-statement,
- comparing the avoidability with a predefined threshold-value indicating whether the mispositioning-type (M) is avoidable or not,
- in the case the comparison shows that the mispositioning-type (M) is avoidable, outputting a predefined dataset allocated to the mispositioning type comprising detailed information on how to avoid such mispositioning in the future.

12. The method according to one of claims 5 to 11, wherein the positioning-advice (A) is designed according to the physical nature of the patient (P), and is especially patient-specific, and/or is designed based on predefined information and/or prior data of the examiner for the medical examination.

13. A medical imaging system, especially a mammography-system (1) or a fluoroscopy-system, comprising a device (10) according to one of claims 1 to 4 and/or designed for performing a method according to one of claims 5 to 12.

14. A computer program product comprising instructions which, when the program is executed by a computer, cause the computer to carry out the steps of the training-method of any of claims 5 to 12.

15. A computer-readable storage medium comprising instructions which, when executed by a computer, cause the computer to carry out the steps of the training-method of any of claims 5 to 12.
